# NEUE EUROPÄISCHE PATENTSCHRIFT

(11) **EP 1 656 122 B2**
(45) Veröffentlichungstag und Bekanntmachung des Hinweises auf die Entscheidung über den Einspruch: **28.11.2012**
(45) Hinweis auf die Patenterteilung: 28.10.2009
(21) Anmeldenummer: 04764129.5
(22) Anmeldetag: 14.08.2004
(51) Int. Cl.: A61K 9/70, A61K 45/06, A61K 31/13, A61K 31/428, A61K 31/381, A61P 25/16

(54) **TRANSDERMALE ARZNEIMITTELZUBEREITUNGEN MIT WIRKSTOFFKOMBINATIONEN ZUR BEHANDLUNG DER PARKINSON-KRANKHEIT**
MEDICAMENT PREPARATIONS FOR TRANSDERMAL APPLICATION CONTAINING ACTIVE INGREDIENT COMBINATIONS FOR TREATING PARKINSON'S DISEASE
PREPARATIONS PHARMACEUTIQUES POUR ADMINISTRATION TRANSDERMIQUE COMPRENANT DES COMBINAISONS DE PRINCIPES ACTIFS, UTILISEES POUR TRAITER LA MALADIE DE PARKINSON

(30) Priorität: 20.08.2003 DE 10338174
(43) Veröffentlichungstag der Anmeldung: 17.05.2006
(73) Patentinhaber: LTS LOHMANN Therapie-Systeme AG, 56626 Andernach (DE)
(72) Erfinder: HORSTMANN, Michael, 56564 Neuwied (DE); THEOBALD, Frank, 53498 Bad Breisig (DE)
(74) Vertreter: Michalski Hüttermann & Partner
(86) Internationale Anmeldenummer: PCT/EP2004/009136
(87) Internationale Veröffentlichungsnummer: WO 2005/018619

(56) Entgegenhaltungen:
- WO-A-02/47666
- WO-A-89/09051
- DE-A- 3 710 966
- FR-A- 2 788 982
- US-A- 5 462 746
- US-A1- 2003 119 884
- REIDERER P, LAUX G, PÖLDINGER W: "Neuro-Psychopharmaka, Bd 5" 1992, SPRINGER VERLAG , WIEN , XP009040582 in der Anmeldung erwähnt das ganze Dokument
- MUCKE H A M: "ROTIGOTINE SCHWARZ PHARMA" IDRUGS, CURRENT DRUGS LTD, GB, Bd. 6, Nr. 9, 2003, Seiten 894-899, XP001157133 ISSN: 1369-7056
- TUITE P ET AL: "RECENT DEVELOPMENTS IN THE PHARMACOLOGICAL TREATMENT OF PARKINSON'S DISEASE" EXPERT OPINION ON INVESTIGATIONAL DRUGS, ASHLEY PUBLICATIONS LTD., LONDON, GB, Bd. 12, Nr. 8, 1. August 2003 (2003-08-01), Seiten 1335-1352, XP009023949 ISSN: 1354-3784
- ABRAMSKY O ET AL: "Combined treatment of Parkinsonian tremor with propranolol and levodopa." JOURNAL OF THE NEUROLOGICAL SCIENCES. DEC 1971, Bd. 14, Nr. 4, Dezember 1971 (1971-12), Seiten 491-494, XP001204127 ISSN: 0022-510X
- CHARLES P D ET AL: "CLASSIFICATION OF TREMOR AND UPDATE ON TREATMENT" AMERICAN FAMILY PHYSICIAN, AMERICAN ACADEMY OF FAMILY PHYSICIANS, US, Bd. 59, Nr. 6, 15. März 1999 (1999-03-15), Seiten 1565-1572, XP000852624
- IRAVANI MAHMOUD M ET AL: "3,4-Methylenedioxymethamphetamine (Ecstasy) inhibits dyskinesia expression and normalizes motor activity in 1-methyl-4-phenyl-1,2,3,6 tetrahydropyridine-treated primates." JOURNAL OF NEUROSCIENCE, Bd. 23, Nr. 27, 8. Oktober 2003 (2003-10-08), Seiten 9107-9115, XP002308002 ISSN: 0270-6474

## Beschreibung

Die Erfindung betrifft transdermal verabreichbare Arzneimittelzubereitungen zur Behandlung des Parkinson-Syndroms, wobei diese Zubereitungen eine Kombination von mindestens zwei für die Parkinson-Therapie geeignete Wirkstoffe enthalten. Die Erfindung betrifft ferner die Verwendung einer solchen Wirkstoffkombination zur Herstellung eines transdermal verabreichbaren Arzneimittels zur Behandlung der Parkinson-Krankheit, sowie die therapeutische Behandlung von Parkinson-Patienten durch transdermale Verabreichung einer der genannten Arzneimittelzubereitungen.

Zur medikamentösen Therapie der Parkinson-Krankheit werden derzeit Wirkstoffe aus folgenden Gruppen eingesetzt: Anti-Parkinson-Mittel mit dopaminerger Wirkung, insbesondere L-Dopa und Dopaminrezeptor-Agonisten; zentral wirksame Anti-cholinergika (Muscarinrezeptor-Antagonisten); Monoaminoxidase-Hemmer; NMDA-Antagonisten; beta-Sympatholytica. Daneben wird auch die Verabreichung von Sympathomimetika aus der Gruppe der Phenylethylamin-Derivate (z. B. Ecstasy = MDMA) in Erwägung gezogen, da bei einzelnen Parkinson-Patienten, die nach langdauernder Anwendung von L-Dopa unter Dyskinesien leiden, eine Besserung der Symptomatik beobachtet wurde.

Wirksamstes und wichtigstes Parkinson-Medikament ist L-Dopa (Levodopa), das fast ausschließlich oral verabreicht wird, bevorzugt in Kombination mit Decarboxylasehemmern (z. B. Benserazid, Carbidopa) oder Inhibitoren der Catechol-O-Methyltransferase (z. B. Entacapon). Allerdings kommt es bei der Langzeit-Anwendung von L-Dopa zu Dyskinesien, Wirkungsfluktuationen und zu einem Wirkungsverlust. Aus diesem Grund wird angestrebt, die Dosierung von L-Dopa möglichst gering zu halten oder zu Beginn der Therapie auf den Einsatz von L-Dopa ganz zu verzichten. Beispielsweise kann der Zeitpunkt für den Beginn einer obligaten L-Dopa-Gabe durch Verabreichung des Monoaminoxidase-Hemmstoffes L-Deprenyl (= Selegilin) hinausgezögert werden. Ebenso kann durch eine Begleittherapie mit Selegilin eine Dosiseinsparung von L-Dopa um 25-30 % ermöglicht werden. Ferner wurde beobachtet, daß insbesondere bei jüngeren Parkinson-Patienten durch frühzeitige Gabe von Selegilin das Auftreten der als sehr lästig empfundenen Dyskinesien unterdrückt werden kann.

Neben L-Dopa werden zur Therapie der Parkinson-Krankheit auch Dopamin-agonistisch wirkende Stoffe wie z. B. Lisurid, Bromocriptin, Pramipexol, Ropirinol, Rotigotin, Tergurid, Cabergolin, Apomorphin, Piribedil, PHNO (4-Propyl-9-hydroxynaphthoxazin) verwendet. Mit solchen dopaminerg wirkenden Stoffen kann der bei Parkinson-Kranken fehlende Transmitter Dopamin weitgehend substituiert werden.

Eine bedeutende Rolle für die Begleittherapie von Parkinson-Kranken kommt der Behandlung mit anticholinerg wirkenden Stoffen (Muscarinrezeptor-Antagonisten) zu, wodurch insbesondere der Tremor unterdrückt werden kann. In dieser Wirkstoffgruppe sind beispielsweise Bipreriden, Trihexyphenidyl, Procyclidin, Bornaprin, Metixen, Orphenadrin, Scopolamin, Atropin und andere Belladonna-Alkaloide, Benzatropin und Nicotin von therapeutischer Bedeutung.

Für die Parkinson-Therapie geeignet ist ferner eine weitere Gruppe von Wirkstoffen, bei denen es sich um NMDA (N-methyl-D-aspartat)-Rezeptorantagonisten handelt; hierzu gehören z. B. Mematin und Amantadin. Diese Stoffe wirken mit L-Dopa synergistisch und ermöglichen es im Falle einer Kombinationstherapie, die L-Dopa-Dosis zu reduzieren. Insbesondere die Akinesie der Parkinson-Kranken kann durch die zusätzliche Gabe von NMDA-Rezeptorantagonisten günstig beeinflußt werden.

Die Kombinationstherapie des Parkinsonismus ist allgemein eingeführt, und die daraus resultierenden vorteilhaften Wirkungen sind bekannt (z. B. E. Schneider, "Kombinierte Therapien"; in: "Neuro-Psychopharmaka", Bd. 5: Parkinsonmittel und Nootropika, S. 131-144; Hrsg. P. Riederer et al.; Springer Verlag, Wien, 1992). Grundsätzlich ist die Kombination oraler Medikamente jedoch nachteilig, da die zu einem bestimmten Zeitpunkt erzielte Wirkung von der eher zufälligen Eigenkinetik des Wirkstoffen nach oraler Resorption als Einzeldosis abhängig ist. Da die in einer Wirkstoffkombination enthaltenen Einzel-Wirkstoffe sich hinsichtlich ihrer Halbwertszeiten und oralen Dosierungsintervalle unterscheiden, kommt es nicht zu einer gleichmäßigen Exposition des Kranken mit den verschiedenen Wirkstoffen, sondern die relativen Wirkungen einer jeden Wirkstoffkomponente schwanken sehr stark, abhängig von den tageszeitlichen Änderungen des Wirkstoffspiegel-Verlaufs.

Aufgrund dieser bekannten pharmakokinetischen Besonderheiten werden "fixe" Wirkstoffkombinationen innerhalb einer oralen Darreichungsform (z. B. Tablette mit Kombinationswirkstoffen) von den Arzneimittelzulassungs-Behörden als sehr problematisch angesehen und nur in Ausnahmefällen zugelassen. Eine Kombinationstherapie wird deshalb in der Regel durch getrennte Verabreichung zweier oder mehrerer Monosubstanzpräparate durchgeführt. Dies erfordert dann aber genau auf die einzelnen Halbwertszeiten dieser Einzelwirkstoffe angepaßte Dosierungsintervalle. Jedoch läßt sich auch in diesem Fall nicht vermeiden, daß die jeweiligen Wirkstoffkonzentrationen der einzelnen Wirkstoffe, insbesondere im Verhältnis zueinander, großen Schwankungen unterliegen. Hinzu kommt, daß die genaue Einhaltung der Dosierungsintervalle durch den Patienten oder das Pflegepersonal nicht immer gewährleistet ist. Durch die Nichteinhaltung des Dosierungsschemas werden die vorteilhaften Wirkungen der Kombinationstherapie unter Umständen erheblich gemindert. Ein weiterer Nachteil der oralen Kombinationstherapie ist, daß durch die gleichzeitige Gabe verschiedener Wirksubstanzen das Risiko von unerwünschten Nebenwirkungen erhöht wird. Diese Nebenwirkungen sind häufig auf das vorübergehende Auftreten von Plasma-Spitzenwerten kurz nach der oralen Verabreichung zurückzuführen.

Neben oralen Parkinson-Medikamenten sind auch transdermal verabreichbare Wirkstoffzubereitungen bekannt, beispielsweise für L-Dopa (Sudo et al.: Transdermal absorption of L-Dopa from hydrogel in rats; Eur. J. Pharm. Sci. 7 (1998), 67-71), Rotigotin (EP-A-1 256 339) und Deprenyl = Selegilin (EP-B-0 404 807).

Die der Erfindung zugrunde liegende Aufgabe bestand darin, pharmazeutische Zubereitungen bereitzustellen, mit denen eine Kombinationstherapie zur Parkinson-Behandlung auf einfache und sichere Weise durchführbar ist, und die es ermöglichen, die oben genannten Nachteile zu vermeiden oder zu vermindern. Der Erfindung lag ferner die Aufgabe zugrunde, Verfahren für die medikamentöse Kombinationntherapie der Parkinson-Krankheit aufzuzeigen.

Überraschenderweise hat sich gezeigt, daß die vorstehend genannten Aufgaben durch transdermale Arzneimittelzubereitungen gemäß Anspruch 1, 3 oder 10 gelöst werden, sowie durch die in den abhängigen Ansprüchen beschriebenen bevorzugten Ausführungsformen.

Gemäß Hauptanspruch enthalten die erfindungsgemäßen transdermalen Arzneimittelzubereitungen eine Kombination von mindestens zwei Wirkstoffen, welche aus den folgenden Wirkstoffgruppen ausgewählt sind:
a) Dopamin-Agonisten und L-Dopa, und
b) Anticholinergika.

Mindestens zwei der Wirkstoffe gehören unterschiedlichen Wirkstoffgruppen an. Unter "Wirkstoffen" werden sowohl die einzelnen Wirkstoff-Verbindungen als solche verstanden (z. B. freie Wirkstoff-Basen), wie auch deren pharmazeutisch akzeptablen Salze und Additions-Salze (z. B. Säure-Additionssalze). Des weiteren bezieht sich der Begriff "Wirkstoff" gegebenenfalls sowohl auf das pharmakologisch wirksame bzw. stärker wirksame Enantiomer, als auch auf das entsprechende racemische Gemisch.

Im Gegensatz zur oralen Verabreichung einer Wirkstoff-Kombination, bei der die relativen Aktivitäten der Kombinationspartner aufgrund unterschiedlicher Halbwertszeiten Schwankungen unterworfen sind, kann bei transdermaler Gabe ein gleichsinniges Ansteigen und Absinken der Plasmaspiegel eingehalten werden. Da bei transdermaler Verabreichung das Auftreten von Planmaspitzenwerten weitgehend vermieden wird und der zeitliche Verlauf der Plasmakonzentrationen im allgemeinen gleichmäßiger ist, sinkt das Nebenwirkungs-Risiko. Durch die gleichmäßigere Kinetik, insbesondere bei der Verwendung von transdermalen therapeutischen Systemen zur kontrollierten Verabreichung der Wirkstoffkombination, werden die vorteilhaften Wirkungen der kombinierten Wirkstoff-Verabreichung mit größerer Sicherheit erreicht als bei oraler Verabreichung. Insbesondere bei der Verwendung von transdermalen therapeutischen Systemen wird die Einhaltung der vorgesehenen Dosierungsschemata erleichtert und sicherer gemacht.

Es wurde überraschenderweise festgestellt, daß die Kombination von Wirkstoffen aus zwei oder mehreren der genannten Wirkstoffgruppen, denen unterschiedliche Wirkmechanismen zugrunde liegen, zu einer überadditiven Kombination von Wirkungen beiträgt, und daß diese vorteilhaften Wirkungen durch eine kombinierte transdermale Gabe erzielt werden können. Dabei werden gleichmäßige und aufeinander abgestimmte Wirkstoffspiegel der einzelnen Wirkstoffkomponenten erhalten, wodurch die Sicherheit der Kombinationstherapie gewährleistet wird.

Ferner ist überraschenderweise jede einstellbare Zweierkombination von Wirkstoffen möglich (nach den im Hauptanspruch genannten Bedingungen), so daß für bestimmte Gruppen von Parkinson-Patienten eine adäquat medikamentöse Therapie ermöglicht werden kann, beispielsweise abhängig vom Krankheitsstadium oder der Art und dem Schweregrad der Symptome.

Die Gruppe der Dopamin-Agonisten umfaßt insbesondere die Wirkstoffe Lisurid, Bromocriptin, Pramipexol, Ropinirol, Rotigotine, Tergurid, Cabergolin, Apomorphin, Piribedil, Pergolid und 4-Propyl-9-hydroxynaphthoxazin (PHNO).

Die Gruppe der Monoaminoxidase-Hemmer besteht vorzugsweise aus Monoaminoxidase-B(MAO-B)-selektiven Hemmstoffen, wobei Selegilin (= L-Deprenyl) besonders bevorzugt verwendet wird. Selegilin wird bevorzugt in Form seines Säureadditionssalzes verwendet, wobei insbesondere die Salze mit Halogenwasserstoffsäuren (z. B. Selegilin-Hydrochlorid) oder mit organischen Säuren (z. B. Selegilin-Citrat) in Betracht kommen. Selegilin ist besonders geeignet, weil es nicht nur ein hochwirksamer, selektiver und irreversibler MAO-B-Hemmstoff ist, sondern zusätzlich die Wiederaufnahme von Dopamin in catecholaminergen zentralen Neuronen hemmt und eine gewisse Schutzwirkung gegen die neurotoxischen Effekte von 6-OH-Dopamin ausübt. Die transdermale Verabreichung ist besonders vorteilhaft, da aufgrund der Umgehung des First-Pass-Metabolismus - im vergleich zu entsprechenden oralen Dosen - deutlich höhere Plasmaspiegel erreicht werden können, bei deutlich verminderten Plasmakonzentrationen der Metaboliten (u. a. L-Amphetamin, Methamphetamin), welche als problematisch eingestuft werden.

Aus der Gruppe der Anticholinergika (Muscarinrezeptor-Antagonisten) kommen folgende Wirkstoffe in Betracht: Bipreriden, Trihexyphenidyl, Procyclidin, Bornaprin, Metixen, Orphenadrin, Scopolamin, Atropin und andere Belladonna-Alkaloide und Benzatropin.

Aus der Gruppe der NMDA-Rezeptor-Antagonisten kommen bevorzugt Memantin und Amantadin in Betracht.
Die Gruppe der Sympathomimetika enthält insbesondere Wirkstoffe aus der Gruppe der Phenylethylamin-Derivate, wobei 3,4-Methylendioxymethamphetamin (= MDMA = "Ecstasy") besonders bevorzugt wird.

Gemäß einer weiteren bevorzugten Ausführungsform ist vorgesehen, daß eine erfindungsgemäße transdermale Arzneimittelzubereitung eine Kombination von mindestens zwei Wirkstoffen enthält, und zwar
a) eine Kombination aus einem Dopamin-Agonisten oder L-Dopa, einem anticholinergisch wirkenden Stoff und einem NMDA-Rezeptor-Antagonisten,
   oder
b) eine Kombination aus einem Dopamin-Agonisten oder L-Dopa, einem anticholinergisch wirkenden Stoff und einem Monoaminoxidase-B-Hemmstoff, insbesondere Selegilin.

Gemäß einer weiteren bevorzugten Ausführungsform der Erfindung können die Arzneimittelzubereitungen Kombination von drei Wirkstoffen enthalten, vorzugsweise
a) eine Kombination aus einem Dopamin-Agonisten oder L-Dopa, einem anticholinergisch wirkenden Stoff und einem NMDA-Rezeptor-Antagonisten,
   oder
b) eine Kombination aus einem Dopamin-Agonisten oder L-Dopa, einem anticholinergisch wirkenden Stoff und einem Monoaminoxidase-B-Hemmstoff, insbesondere Selegilin.

Bei einer derartigen Dreier-Kombination ist der Dopamin-Agonist für die Grund-Therapie der Parkinson-Krankheit verantwortlich, während der anticholinergisch wirkende Stoff den Tremor günstig beeinflussen kann und mit dem NMDA-Rezeptor-Antagonisten eine zusätzliche Verbesserung der Akinesie erreicht werden kann. Die (zusätzliche) Gabe von Selegilin hat gleichzeitig positive Effekte auf die Verhinderung des Fortschreitens der Krankheit, außerdem wird die erforderliche Dopamin-Agonist-Dosis vermindert.

Gemäß einer weiteren bevorzugten Ausführungsform enthält eine erfindungsgemäße Arzneimittelzubereitung eine Kombination von Selegilin mit einem Dopamin-Agonisten aus der Ropirinol, Pramipexol und Rotigotin umfassenden Gruppe. Die Kombination aus Selegilin und Rotigotin wird dabei besonders bevorzugt.

Des weiteren ist es vorteilhaft, wenn eine der vorstehend beschriebenen Arzneimittelzubereitungen, insbesondere eine L-Dopa enthaltende Kombination, zusätzlich mindestens einen weiteren Wirkstoff enthält, der aus der Gruppe ausgewählt ist, die Catechol-o-Methyltransforase-Hemmer und Decarboxylase-Hemmer umfaßt, wobei Entacapon, Benserazid und Carbidopa besonders bevorzugt sind.

Insbesondere zur Behandlung des Parkinson-bedingten Tremors kann es vorteilhaft sein, wenn eine erfindungsgemäße Arzneimittelzubereitung zusätzlich mindestens einen Wirkstoff aus der Gruppe der Beta-Blocker enthält, vorzugsweise aus der Propranolol, Timolol, Pindolol und Atenolol umfassenden Gruppe.

Die erfindungsgemäßen transdermalen Arzneizubereitungen sind als transdermale therapeutische Systeme (TTS) formuliert welche eine transdermale, systemische Wirkstoffverabreichung ermöglichen; der Aufbau solcher Systeme und die hierfür geeigneten Formulierungshilfsstoffe und sonstigen Materialien sind dem Fachmann bekannt. Transdermale therapeutische Systeme ermöglichen die kontrollierte Abgabe von Wirkstoffen an die Haut, mit vorherbestimmbarer Freisetzungsrate innerhalb eines gegebenen Zeitraums.

Es hat sich gezeigt, daß TTS aufgrund dieser speziellen Eigenschaften besonders für die Verabreichung von Wirkstoffkombinationen bei der Behandlung von Parkinson-Kranken geeignet sind, da der zeitliche Verlauf der Plasmaspiegel der einzelnen Wirkstoffkamponenten wesentlich gleichmäßiger ist als bei oraler Verabreichung und demzufolge geringere Nebenwirkungen verursacht werden. Zudem wird durch die Verwendung von Wirkstoffkombinations-TTS das Problem der Einhaltung der Dosierungsintervalle erheblich verringert, zum einen, weil durch Applikation eines einzigen TTS zwei oder mehrere Wirkstoffe zusammen verabreicht werden können, zum andern, weil ein TTS im typischen Fall über einen Applikationszeitraum von ca. 6 bis 48 h auf der Haut des Patienten verbleiben kann. Beispielsweise könnte eine Behandlung in der Weise erfolgen, daß ein Wirkstoffkombinations-TTS einmal täglich oder an jedem zweiten Tag erneuert wird. Dadurch wird die Anwendung erheblich vereinfacht, verglichen mit der oralen Kombinationntherapie mit Monosubstanzpräparaten.

Die erfindungsgemäßen TTS sind als auf der Haut haftklebende Wirkstoffpflaster formuliert; die Wirkstoffkombination ist, gegebenenfalls mit weiteren Hilfsstoffen, in einem Wirkstoffreservoir enthalten, das entweder vom Matrix-Typ ist oder beutelförmig gestaltet ist. Der Aufbau der TTS umfaßt zusätzlich eine wirkstoffundurchlässige Rückschicht sowie eine ebenfalls wirkstoffundurchlässige, abziehbare Schutzfolie.

Das erwähnte beutelförmige Reservoir ist mit einer flüssigen, hochviskosen, halbfesten oder thixotropen Matrix gefüllt; insbesondere kann es als Gel formuliert sein. Die der Haut abgewandte Beutelrtickseite muß dabei wirkstoffundurchlässig, die der Haut zugewandte Seite wirkstoffdurchlässig sein. Optional kann eine wirkstoffdurchlässige Membran (Steuermembran) die Steuerung der Wirkstofffreisetzung übernehmen. Hierfür geeignete Materialien und Hilfsstoffe sind dem Fachmann bekannt.

Besonders bevorzugt sind TTS, bei denen die Wirkstoffkombination in einer festen Matrix enthalten ist. Im einfachsten Fall kann ein erfindungsgemäßes TTS erhalten werden, indem eine der beschriebenen Wirkstoffkombinationen in einer Lösung von Matrix-Grundpolymeren grob (d. h. partikulär), kolloidal oder molekular dispergiert bzw. gelöst wird, und diese Mischung auf eine geeignete Unterlage - in der Regel eine silikonisierte thermoplastische Folie (die spätere Schutzschicht) - beschichtet wird. Nach Trocknen bzw. Abdampfen der Lösemittelanteile wird die erhaltene Schicht, welche das haftklebende wirkstoffreservoir darstellt, mit einer weiteren Folie abgedeckt, welche die spätere Rückschicht des TTS darstellt. Aus diesem Laminat können anschließend durch Stanzen flächiger Gebilde TTS in der gewünschten geometrischen Form erhalten werden.

Als Rückschicht eignen sich vor allem Polyester, darüber hinaus aber auch nahezu beliebige andere hautverträgliche Kunststoffe, wie z. B. Polyvinylchlorid, Ethylenvinylacetat, Vinylacetat, Polyethylen, Polypropylen, Cellulosederivate und viele andere mehr. Im Einzelfall kann die Rückschicht mit einer zusätzlichen Auflage versehen werden, z. B. durch Bedampfung mit Metallen oder anderen diffusionssperrenden Zusatzstoffen wie Siliciumdioxid, Aluminiumoxid oder ähnlicher Stoffe, die dem Fachmann bekannt sind. Für die ablösbare Schutzfolie können dieselben Materialien verwendet werden wie für die Rückschicht, vorausgesetzt, daß sie durch geeignete Oberflächenbehandlung, wie z. B. Silikonisierung, ablösbar ist.

Geeignete Basis-Polymere für die Herstellung des/der Matrixschicht(en) sind vor allem Polymere auf Basis von Acrylsäure und deren Ester, Polyacrylaten, Isobutylen, EthylenVinylacetat, Kautschuken, Mischungen aus Kautschuken und Harzen, Cellulose-Derivaten, insbesondere Methyl- und Ethylcellulosen, Styrol-Dien-Copolymeren, Synthesekautschuken, Silikon-Haftklebern oder Heißschmelzklebern. Mit Vorteil können auch geeignete Mischungen der genannten Polymere zum Einsatz kommen. Unter den Begriff "Heißschmelzkleber" fallen alle Kleber, die nicht durch Lösemittel, sondern durch Schmelzen bei erhöhten Temperaturen, beispielsweise im Bereich von 60-200 °C verflüssigt werden. Als Heißschmelzkleber eignen sich z. B. Mischungen aus Estern des hydrierten Kolophoniums mit Cellulosederivaten.

Die erfindungsgemäßen TTS können optional weitere Hilfsstoffe enthalten, insbesondere aus den Gruppen der Hautpermeationaverstärker, Weichmacher, Klebrigmacher, pH-Regulatoren und Antioxidantien.
Als permeationsfördernde Stoffe eignen sich vor allem Stoffe aus den Gruppen der Fettalkohole, Fettsäuren, Polyoxyethylenfettalkoholether, Polyoxyethylenfettsäureester, Fettalkoholester und Fettsäureester, insbesondere Sorbitanmonolaurat oder Ester von langkettigen Fettsäuren mit Methyl-, Ethyl- oder Isopropylalkohol, oder Ester von Fettalkoholen mit Essigsäure oder Milchsäure. Auch Stoffe wie Ölsäurediethanolamin kommen in Betracht. Der Mengenanteil dieser Stoffe beträgt 0,1 bis 25 Gew.-%, vorzugsweise von 1 bis 10 Gew.-%, jeweils bezogen auf das Gesamtgewicht der Wirkstoffmatrix.

Bei der Herstellung von TTS, welche eine bevorzugte Wirkstoffkombination mit dem Wirkstoff Selegilin enthalten, kann nach den in den US-Patenten 5,462,746 und 5,902,601 beschriebenen Verfahren vorgegangen werden. Selegilin-Base ist flüssig und bei Raumtemperatur leicht flüchtig, wodurch die Herstellung von TTS erschwert wird. Das nicht flüchtige Hydrochlorid des Selegilin ist wegen der schlechteren Hautgängigkeit weniger geeignet. Nach US 5,462,746 wird Selegilin-Hydrochlorid mit einer Lösung der Matrix-Grundpolymere (z. B. Haftkleberpolymere) vermischt und diese Mischung auf eine Unterlage beschichtet. Die getrocknete Schicht wird mit einer zweiten Matrix-schicht bedeckt, die basische Gruppen enthält, welche in der Lage sind, die freie Base aus dem Wirkstoffsalz freizusetzen.
Nach US 5,902,601 (oder DE-A 43 32 094) werden die Matrix-Grundpolymere in dem flüssigen, leicht flüchtigen Wirk- oder Hilfsstoff gelöst und auf eine Unterlage beschichtet. Auf diese Schicht werden eine oder mehrere weitere Matrixschichten laminiert, welche keinen derartigen leicht flüchtigen Stoff enthalten. Durch Migration des Wirk- oder Hilfsstoffs in die weitere(n) Matrixschicht(en) wird eine insgesamt scherstabile Wirkstoffmatrix erhalten.
Die Erfindung erfaßt somit auch TTS, die durch Laminieren von mindestens zwei Schichten, welche jeweils mindestens einen Wirkstoff enthalten, hergestellt sind, wobei für die Herstellung einer ersten Schicht ein leicht flüchtiger Wirk- oder Hilfsstoff (z. B. Selegilin-Base) als Lösemittel für das Matrixgrundmaterial verwendet wird, und wobei diese Schicht auf eine zweite Schicht laminiert wird, welche ohne Verwendung eines leicht flüchtigen Wirk- oder Hilfsstoffes hergestellt ist, und wobei durch die diffusive Migration des leicht flüchtigen Wirk- oder Hilfsstoffes in die genannte zweite Schicht ein scherstabiler Verbund und eine einheitlich erscheinende Matrix erhalten wird.

Die genannten mindestens zwei Wirkstoffe der Wirkstoffkombination sind in unterschiedlichen Schichten oder Kompartimenten des TTS enthalten.

Die erfindungsgemäßen TTS weisen vorzugsweise eine Flächengröße in Bereich von 5 bis 50 cm² auf; der gesamte Wirkstoffgehalt, bezogen auf das wirkstoffhaltige Reservoir, beträgt vorzugsweise 0,1 bis 50 Gew.-%, bevorzugt 1 bis 10 Gew.-%. Die Wirkstoff-Freisetzungsrate beträgt vorzugsweise mindestens 0,1 mg/cm²d; die freigesetzte Tagesdosis, bezogen auf die Wirkstoffkombination, liegt im Bereich von ca. 0,1 mg bis 50 mg. Freisetzungsrate und Tagesdosis können für die einzelnen Komponenten unterschiedlich hoch eingestellt sein, je nach beabsichtigter therapeutischer Wirkung.
Die erfindungsgemäßen TTS ermöglichen die kontrollierte, gleichbleibende Abgabe einer Wirkstoffkombination über einen Zeitraum im Bereich von vorzugsweise 0,5 bis 7 Tagen, insbesondere 1 bis 3 Tagen.

Des weiteren werden durch die Erfindung auch Verfahren zur therapeutischen Behandlung von Parkinson-Patienten aufgezeigt; diese Verfahren beruhen darauf, daß einer von dieser Krankheit betroffenen Person eine Wirkstoffkombination, wie oben definiert, auf transdermalem Wege in Form eines TTS verabreicht wird, welche in bestimmten Zeitintervallen (z. B. 2-mal täglich, täglich, an jedem 2. Tag, usw.) erneuert werden.

Durch die vorliegende Erfindung wird die Kombinationstherapie des Parkinson-Syndroms einfacher und sicherer gemacht, und es werden zusätzliche und vielfältigere Anwendungsmöglichkeiten eröffnet.

## Patentansprüche

1. Transdermale Arzneimittelzubereitung zur Behandlung der Parkinson-Krankheit, enthaltend eine Kombination von mindestens zwei Wirkstoffen, **dadurch gekennzeichnet, daß** die Arzneizubereitung
- eine Kombination aus einem Dopamin-Agonisten und einem anticholinergisch wirkenden Stoff umfaßt, wobei der anticholinergisch wirkende Stoff aus der Trihexyphenidyl, Procyclidin, Bornaprin, Metixen, Orphenadrin, Scopolamin, Atropin und andere Belladonna-Alkaloide und Benzatropin bestehenden Gruppe ausgewählt ist; oder
- eine Kombination aus L-Dopa und einem anticholinergisch wirkenden Stoff, wobei der anticholinergisch wirkende Stoff aus der Biperiden, Trihexyphenidyl, Procyclidin, Bornaprin, Metixen, Orphenadrin, Scopolamin, Atropin und andere Belladonna-Alkaloide, Benzatropin und Nicotin bestehenden Gruppe ausgewählt ist,
wobei die Arzneimittelzubereitung als transdermales therapeutisches System in Form eines auf der Haut klebenden Wirkstoffpflasters vorliegt, und die genannten mindestens zwei Wirkstoffe in unterschiedlichen Schichten oder Kompartimenten des transdermalen therapeutischen Systems enthalten sind.

2. Arzneimittelzubereitung nach Anspruch 1, **dadurch gekennzeichnet, daß** sie eine Kombination von drei Wirkstoffen enthält, nämlich:
- eine Kombination aus einem Dopamin-Agonisten oder L-Dopa, einem anticholinergisch wirkenden Stoff und einem NMDA-Rezeptor-Antagonisten; oder
- eine Kombination aus einem Dopamin-Agonisten oder L-Dopa, einem anticholinergisch wirkenden Stoff und einem Monoaminoxidase-B-Hemmstoff.

3. Transdermale Arzneimittelzubereitung zur Behandlung der Parkinson-Krankheit, enthaltend eine Kombination von mindestens zwei Wirkstoffen, **dadurch gekennzeichnet, daß** die Arzneizubereitung eine Kombination von drei Wirkstoffen enthält, nämlich
- eine Kombination aus einem Dopamin-Agonisten oder L-Dopa, einem anticholinergisch wirkenden Stoff und einem NMDA-Rezeptor-Antagonisten; oder
- eine Kombination aus einem Dopamin-Agonisten oder L-Dopa, einem anticholinergisch wirkenden Stoff und einem Monoaminoxidase-B-Hemmstoff,
wobei die Arzneimittelzubereitung als transdermales therapeutisches System in Form eines auf der Haut klebenden Wirkstoffpflasters vorliegt, und die genannten mindestens zwei Wirkstoffe in unterschiedlichen Schichten oder Kompartimenten des transdermalen therapeutischen Systems enthalten sind.

4. Arzneimittelzubereitung nach einem der Ansprüche 1 bis 3, **dadurch gekennzeichnet, daß** die Gruppe der Dopamin-Agonisten Lisurid, Bromocriptin, Pramipexol, Ropinirol, Rotigotine, Tergurid, Cabergolin, Apomorphin, Piribedil, Pergolid und 4-Propyl-9-hydroxynaphthoxazin (PHNO) umfaßt.

5. Arzneimittelzubereitung nach einem der Ansprüche 2 bis 4, **dadurch gekennzeichnet, daß** die Gruppe der Monoaminoxidase Hemmer aus Monoaminoxidase-B-selektiven Hemmstoffen besteht, wobei Selegelin besonders bevorzugt ist.

6. Arzneimittelzubereitung nach einem der Ansprüche 3 bis 5, **dadurch gekennzeichnet, dass** die Gruppe der Anticholinergika folgende Wirkstoffe umfaßt: Biperiden, Trihexyphenidyl, Procyclidin, Bornaprin, Metixen, Orphenadrin, Scopolamin, Atropin und andere Belladonna-Alkaloide, Benzatropin und Nicotin.

7. Arzneimittelzubereitung nach einem der vorangehenden Ansprüche, **dadurch gekennzeichnet, daß** die Gruppe der NMDA-Rezeptor-Antagonisten Memantin und Amantadin umfaßt.

8. Arzneimittelzubereitung nach einem der vorangehenden Ansprüche, **dadurch gekennzeichnet, daß** die Arzneizubereitung zusätzlich mindestens einen weiteren Wirkstoff enthält, der aus der Catechol-O-Methyltransferase-Hemmer und Decarboxylase-Hemmer umfassenden Gruppe ausgewählt ist, wobei Entacapon, Benserazid und Carbidopa besonders bevorzugt sind.

9. Arzneimittelzubereitung nach einem der vorangehenden Ansprüche, **dadurch gekennzeichnet, daß** sie zusätzlich mindestens einen Wirkstoff aus der Gruppe der Beta-Blocker enthält, vorzugsweise aus der Propanolol, Timolol, Pindolol und Atenolol umfassenden Gruppe.

10. Transdermale Arzneimittelzubereitung, zur Behandlung der Parkinson-Krankheit, **dadurch gekennzeichnet, daß** sie Selegelin und Rotigotin enthält, wobei die Arzneimittelzubereitung als transdermales therapeutisches System in Form eines auf der Haut klebenden Wirkstoffpflasters vorliegt, und die genannten mindestens zwei Wirkstoffe in unterschiedlichen Schichten oder Kompartimenten des transdermalen therapeutischen Systems enthalten sind.

## Claims

1. Transdermal pharmaceutical preparation for the treatment of Parkinson's disease containing a combination of at least two active substances, **characterised in that** said pharmaceutical preparation
- comprises a combination of a dopamine agonist and an anti-cholinergically active substance, wherein the anti-cholinergically active substance is selected from the group consisting of trihexyphenidyl, procyclidine, bornaprine, metixene, orphenadrine, scopolamine, atropine and other belladonna alkaloids, and benzatropine; or
- a combination of L-dopa and an anticholinergically active substance, wherein the anti-cholinergically active substance is selected from the group consisting of biperiden, trihexyphenidyl, procyclidine, bornaprine, metixene, orphenadrine, scopolamine, atropine and other belladonna alkaloids, benzatropine and nicotine,
wherein said pharmaceutical preparation is present as a transdermal therapeutic system in form of an active substance patch adhering to the skin, and the said at least two active substances are contained in different layers or compartments of the transdermal therapeutic system.

2. Pharmaceutical preparation according to claim 1, **characterised in that** it contains a combination of three active substances, namely:
- a combination of a dopamine agonist or L-dopa, an anticholinergically active substance, and an NMDA receptor antagonist; or
- a combination of a dopamine agonist or L-dopa, an anticholinergically active substance, and a monoamine oxidase B inhibitor.

3. Transdermal pharmaceutical preparation for the treatment of Parkinson's disease containing a combination of at least two active substances, **characterised in that** said pharmaceutical preparation contains a combination of three active substances, namely:
- a combination of a dopamine agonist or L-dopa, an anticholinergically active substance, and an NMDA receptor antagonist; or
- a combination of a dopamine agonist or L-dopa, an anticholinergically active substance, and a monoamine oxidase B inhibitor,
wherein said pharmaceutical preparation is present as a transdermal therapeutic system in form of an active substance patch adhering to the skin, and the said at least two active substances are contained in different layers or compartments of the transdermal therapeutic system.

4. Pharmaceutical preparation according to any one of claims 1 to 3, **characterised in that** the group of dopamine agonists comprises lisuride, bromocriptine, pramipexole, ropinirole, rotigotine, terguride, cabergoline, apomorphine, piribedile, pergolide and 4-propyl-9-hydroxynaphthoxazine (PHNO).

5. Pharmaceutical preparation according to any one of claims 2 to 4, **characterised in that** the group of monoamine oxidase inhibitors consists of monoamine oxidase B-selective inhibitors, with selegeline being particularly preferred.

6. Pharmaceutical preparation according to any one of claims 3 to 5, **characterised in that** the group of anticholinergics comprises the following active substances: biperiden, trihexyphenidyl, procyclidine, bornaprine, metixene, orphenadrine, scopolamine, atropine and other belladonna alkaloids, benzatropine and nicotine.

7. Pharmaceutical preparation according to any one of the preceding claims, **characterised in that** the group of NMDA receptor antagonists comprises memantine and amantidine.

8. Pharmaceutical preparation according to any one of the preceding claims, **characterised in that** said pharmaceutical preparation additionally contains at least one further active substance selected from the group comprising catechol-O-methyl transferase inhibitors and decarboxylase inhibitors, with entacapone, benserazide and carbidopa being particularly preferred.

9. Pharmaceutical preparation according to any one of the preceding claims, **characterised in that** said pharmaceutical preparation additionally contains at least one active substance from the group of the beta blockers, preferably from the group comprising propanolol, timolol, pindolol and atenolol.

10. Transdermal pharmaceutical preparation for the treatment of Parkinson's disease, **characterised in that** it contains selegeline and rotigotine, wherein said pharmaceutical preparation is present as a transdermal therapeutic system in form of an active substance patch adhering to the skin, and the said at least two active substances are contained in different layers or compartments of the transdermal therapeutic system.

## Revendications

1. Préparation médicamenteuse transdermique pour le traitement de la maladie de Parkinson, contenant une combinaison d'au moins deux substances actives, **caractérisée en ce que** la préparation médicamenteuse contient
- une combinaison d'un agoniste de dopamine et d'un substance à effet anticholinergique, où d'une substance à effet anticholinergique est choise dans le groupe comprenant le trihexyphénidyle, la procyclidine, la bornaprine, le metixene, l'orphénadrine, la scopolamine, l'atropine et d'autres alcaloïdes de la belladonne, et le benzatropine;
- une combinaison de L-Dopa et d'une substance à effet anticholinergique, où d'une substance à effet anticholinergique est choise dans le groupe comprenant le bipéridène, le trihexyphénidyle, la procyclidine, la bornaprine, le metixene, l'orphénadrine, la scopolamine, l'atropine et d'autres alcaloïdes de la belladonne, le benzatropine et la nicotine; ,
où la préparation médicamenteuse se trouve sous forme de système thérapeutique transdermique, de forme d'un emplâtre à substance active collant sur la peau, et lesdites au moines deux substances actives sont contenues dans des couches différentes ou des compartiments différents du système thérapeutique transdermique.

2. Préparation médicamenteuse selon la revendication 1, caractérisée en ce que'elle une combinaison de trois substances actives, notamment :
- une combinaison d'un agoniste dopamine ou le L-Dopa, d'une substance à effet anticholinergique et d'un antagoniste du récepteur de NMDA ; ou
- une combinaison d'un agoniste dopamine ou le L-Dopa, d'une substance à effet anticholinergique et d'un inhibiteur de la monoaminoxidase B.

3. Préparation médicamenteuse transdermique pour le traitement de la maladie de Parkinson, contenant une combinaison d'au moins deux substances actives, **caractérisée en ce que** la préparation médicamenteuse contient une combinaison de trois substances actives, notamment :
- une combinasion d'un agoniste dopamine ou le L-Dopa, d'une substance à effet anticholinergique et d'un antagoniste du récepteur de NMDA ; ou
- une combinasion d'un agoniste dopamine ou le L-Dopa, d'une substance à effet anticholinergique et d'un inhibiteur de la monoaminoxidase B,
où la préparation médicamenteuse se trouve sous forme de système thérapeutique transdermique, de forme d'un emplâtre à substance active collant sur la peau, et lesdites au moines deux substances actives sont contenues dans des couches différentes ou des compartiments différents du système thérapeutique transdermique.

4. Préparation médicamenteuse selon l'une quelconque des revendications 1 à 3, **caractérisée en ce que** le groupe des agonistes de la dopamine comprend le lisuride, la bromocriptine, le pramipexol, le ropinirol, la rotigotine, le terguride, la cabergoline, l'apomorphine, le piribedil, le pergolide et la 4-propyl-9-hydroxynaphthoxyzine (PHNO).

5. Préparation médicamenteuse selon l'une quelconque des revendications 2 à 4, **caractérisée en ce que** le groupe des inhibiteurs de la monoaminoxydase est constitué par des inhibiteurs sélectifs de la monoaminoxydase-B. où on préfère en particulier la sélégiline.

6. Préparation médicamenteuse selon l'une quelconque des revendications 3 à 5, **caractérisée en ce que** le groupe des anticholinergiques comprend les substances actives suivantes : e bipéridène, le trihexyphénidyle, la procyclidine, la bornaprine, le metixene, l'orphénadrine, la scopolamine, l'atropine et d'autres alcaloïdes de la belladonne, le benzatropine et la nicotine.

7. Préparation médicamenteuse selon l'une quelconque des revendications précédentes, **caractérisée en ce que** le groupe des antagonistes du récepteur de NMDA comprend la memantine et l'amantadine.

8. Préparation médicamenteuse selon l'une quelconque des revendications précédentes, **caractérisée en ce que** la préparation médicamenteuse contient en outre au moins une autre substance active, qui est choisie dans le groupe comprenant les inhibiteurs de la catéchol-O-méthyltransférase et les inhibiteus de la décarboxylase, où on préfère en particulier l'entacapon, le bensérazide et le carbidopa.

9. Préparation médicamenteuse selon l'une quelconque des revendications précédentes, **caractérisée en ce qu'**elle continent en outre au moins une substance active du groupe des bêta-bloquants, de préférence du groupe comprenant le propanolol, le timolol, le pindolol et l'aténolol.

10. Préparation médicamenteuse transdermique destinée au traitement de la maladie de Parkinson, **caractérisée en ce qu'**elle contient de la sélégiline et de la rotigotine, où la préparation médicamenteuse se trouve sous forme de système thérapeutique transdermique, de forme d'un emplâtre à substance active collant sur la peau, et lesdites au moines deux substances actives sont contenues dans des couches différentes ou des compartiments différents du système thérapeutique transdermique.
